(19) **Europäisches Patentamt**
**European Patent Office**
**Office européen des brevets**

(11) **EP 3 643 376 A1**

(12) **EUROPÄISCHE PATENTANMELDUNG**

(43) Veröffentlichungstag:
**29.04.2020 Patentblatt 2020/18**

(21) Anmeldenummer: **18202307.7**

(22) Anmeldetag: **24.10.2018**

(51) Int Cl.:
*B01D 3/32* (2006.01)  *B01D 3/42* (2006.01)
*C07C 263/20* (2006.01)  *G05D 21/00* (2006.01)
*G05B 13/04* (2006.01)

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Covestro Deutschland AG**
**51373 Leverkusen (DE)**

(72) Erfinder: **Die Erfindernennung liegt noch nicht vor**

(74) Vertreter: **Levpat**
**c/o Covestro AG**
**Gebäude 4825**
**51365 Leverkusen (DE)**

(54) **THERMISCHES TRENNVERFAHREN MIT SOFT-SENSOR**

(57)    Die vorliegende Erfindung betrifft ein Verfahren zur thermischen Trennung einer Mischung umfassend eine erste Hauptkomponente und eine zweite Hauptkomponente, wobei der Siedepunkt der ersten Hauptkomponente niedriger als der Siedepunkt der zweiten Hauptkomponente ist. Die Erfindung betrifft weiterhin ein System zur thermischen Trennung umfassend einen Rechner zur Steuerung der thermischen Trennung, welcher zur Steuerung des erfindungsgemäßen Verfahrens eingerichtet ist. Über vorbestimmte thermodynamische Modelle werden aus Druck- und Temperaturdaten die Anteile von erster und zweiter Hauptkomponente in Sumpf- produktströmen ermittelt.

FIG. 1

**Beschreibung**

Die vorliegende Erfindung betrifft ein Verfahren zur thermischen Trennung einer Mischung umfassend eine erste Hauptkomponente und eine zweite Hauptkomponente, wobei der Siedepunkt der ersten Hauptkomponente niedriger als der Siedepunkt der zweiten Hauptkomponente ist. Die Erfindung betrifft weiterhin ein System zur thermischen Trennung umfassend einen Rechner zur Steuerung der thermischen Trennung, welcher zur Steuerung des erfindungsgemäßen Verfahrens eingerichtet ist.

Verfahren zur Echtzeitüberwachung, Einschätzung und Unterstützung der Entscheidungsfindung in chemischen Anlagen sind beispielsweise in WO 2014/175962 A1 bekannt. Offenbart wird ein Echtzeitverfahren zum Betreiben einer Anlage, welche ein chemisches Verfahren durchführt. Das Verfahren umfasst das kontinuierliche oder intermittierende Gewinnen von einem oder mehreren Messwerten für das Verfahren. Optional werden eine oder mehrere abgeleitete Prozessvariablen kontinuierlich, periodisch oder intermittierend aus gemessenen Verfahrensvariablen und/oder mathematischen Modellen geschätzt. Der momentane Zustand des chemischen Verfahrens wird auf Basis der Prozessvariablen-Messungen und/oder abgeleiteten Prozessvariablen abgeschätzt und der Momentanzustand wird beurteilt. Wahrscheinliche zukünftige Verfahrenszustände werden in die Zukunft projiziert und der momentane und/oder zukünftige wahrscheinliche Zustände werden mit Informationen in einer Datenbank verknüpft. Die Informationen umfassen bevorzugte Aktionen zur vorteilhaften Beeinflussung des zukünftigen Verfahrenszustandes. Mitarbeitern, die die Anlage betreiben, werden die Informationen bereitgestellt. Optional werden manuelle und/oder automatische Handlungen durchgeführt, die den zukünftigen Verfahrenszustand vorteilhaft beeinflussen, durchgeführt.

Eine wichtige Größe zur effektiven Fahrweise im HDI-Prozess zur Herstellung von 1,6-Hexamethylendiisocyanat (HDI) ist die Einstellung der Konzentration von Monochlorbenzol (MCB). Die MCB-Konzentration hat einen großen Einfluss auf den HDI-Prozess. Weiterhin ist es vorteilhaft, eine konstante Fahrweise des MCB-Kreislaufs anzustreben, zum Beispiel über Eingriffe des Anlagenfahrers oder eine Automatisierung, so dass der MCB-Kreislauf als Teil des HDI-Prozesses optimiert werden kann.

Für eine Regelung (über Eingriffe des Anlagenfahrers bzw. eine Automatisierung) des MCB-Kreislaufs im HDI-Prozess wäre eine zeitnahe Erfassung der MCB-Konzentration wünschenswert. Üblicherweise wird hierzu die Konzentration nach einer Probennahme mittels Gas-Chromatographie in einem Labor bestimmt. Diese Messungen erfolgen in zeitlich eher großen Abständen und haben zusätzlich eine Verzögerung von der Probennahme bis zur Analyse von mehreren Stunden.

Eine indirekte Erfassung der MCB-Konzentration über eine Temperaturmessung an nur einer Messstelle, an der ein Flüssig-Dampf-Gleichgewicht (vapor-liquid equilibrium, VLE) vorliegt, ist zwar grundsätzlich möglich, da die Komponenten (MCB, HDI) die Stoffzusammensetzung dominieren und alle weiteren Komponenten nur mit einer kleinen Konzentration vorkommen oder aber eine höhere Siedetemperatur haben als der Schwersieder des betrachteten binären Gemisches, so dass die weiteren Komponenten effektiv keinen Partialdruck beitragen. Voraussetzung für diese Vorgehensweise ist ein konstanter Druck. Allerdings verändert sich der Druck im HDI-Prozess aufgrund unterschiedlicher Betriebsbedingungen, so dass die MCB-Konzentration trotz Temperaturregelung nicht konstant gefahren wird. Daher scheidet diese Vorgehensweise in der Praxis aus.

Wünschenswert für die Regelung des Prozesses ist eine kontinuierliche Bestimmung der MCB-Konzentration mit der Abtastzeit des Prozessleitsystems (PLT, Abtastung typischerweise jede Sekunde). Dies gilt für eine gezielte Einstellung eines Betriebspunkts (mit einer Vorgabe des Sollwerts für die MCB-Konzentration) durch den Anlagenfahrer als auch für eine zügige Ausregelung von Störungen. Zusätzlich wünschenswert ist es über eine Massenbilanz und eine Energiebilanz über den Verdampfer und dem Verhältnis der Verdampfungsenthalpien der ersten Komponente und des Dampfstroms die Temperatur des Verdampfers oder der Trenneinheit zu berechnen. Dies hat den Vorteil, dass auf den Einbau eines Messsensors verzichtet werden kann.

Die vorliegende Erfindung hat sich die Aufgabe gestellt, ein Verfahren und ein System bereitzustellen, mit dem insbesondere die MCB-Konzentration im Kontext der HDI-Herstellung zuverlässiger und schneller bestimmt werden kann, ein gewünschter Sollwert eingehalten werden kann und diesen Sollwert auch in Gegenwart von Störungen gehalten werden kann.

Gelöst wird diese Aufgabe durch ein Verfahren gemäß Anspruch 1 und ein System gemäß Anspruch 15. Vorteilhafte Weiterbildungen sind in den Unteransprüchen angegeben. Sie können beliebig kombiniert werden, sofern sich aus dem Kontext nicht eindeutig das Gegenteil ergibt.

Ein Verfahren zur thermischen Trennung einer Mischung umfassend eine erste Hauptkomponente und eine zweite Hauptkomponente, wobei der Siedepunkt der ersten

Hauptkomponente niedriger als der Siedepunkt der zweiten Hauptkomponente ist (jeweils unter den gleichen Bedingungen), umfasst somit die Schritte:

A) Verdampfen einer Mischung der ersten Hauptkomponente und der zweiten Hauptkomponente in einem Verdampfer durch Zufuhr von thermischer Energie unter Erhalt einer gasförmigen Mischung der ersten Hauptkomponente und der zweiten Hauptkomponente und eines Sumpfprodukts, welche sich in einem Flüssig-Dampf-Gleichgewicht miteinander befinden;

B) Überführen der gasförmigen Mischung aus Schritt A) in eine thermische Trennvorrichtung, wobei in der Trennvorrichtung die zweite Hauptkomponente wenigstens teilweise als Sumpfprodukt kondensiert, die erste Hauptkomponente wenigstens teilweise in der Gasphase verbleibt und wobei ein Flüssig-Dampf-Gleichgewicht zwischen dem Sumpfprodukt und der Gasphase herrscht;

C) Entfernen des flüssigen Sumpfprodukts aus dem Verdampfer in einem ersten Sumpfproduktstrom mit einem Massenstrom $F_1$;

D) Entfernen des flüssigen Sumpfprodukts aus der Trennvorrichtung in einem zweiten Sumpfproduktstrom mit einem Massenstrom $F_2$;

E) Vereinigen des ersten und des zweiten Sumpfproduktstroms zu einem dritten Sumpfproduktstrom mit einem Mischungsverhältnis $v = F_1/(F_1 + F_2)$;

F) Aufteilen des dritten Sumpfproduktstroms in wenigstens einen Zielproduktstrom mit einem Massenstrom $F_3$ und einen Recycle-Strom mit einem Massenstrom $F_{rec}$, wobei der Zielproduktstrom entnommen wird und der Recycle-Strom in den Verdampfer zurückgeführt wird und wobei der Zielproduktstrom einen Zielwert für die Anteile der ersten und zweiten Hauptkomponente aufweist.

In dem Verdampfer und/oder der Trennvorrichtung wird der dort gemeinsam herrschende Druck p bestimmt und in dem Verdampfer wird die dort herrschende Temperatur $T_1$ und in der Trennvorrichtung die dort herrschende Temperatur $T_2$ bestimmt.

Aus p und $T_1$ werden über ein erstes vorbestimmtes thermodynamisches Modell die Anteile der ersten und zweiten Hauptkomponente in dem ersten Sumpfproduktstrom, ausgedrückt als Qualität $Q_1$, bestimmt, aus p und $T_2$ über ein zweites vorbestimmtes thermodynamisches Modell die Anteile der ersten und zweiten Hauptkomponente in dem zweiten Sumpfproduktstrom, ausgedrückt als Qualität $Q_2$, bestimmt und aus den Qualitäten $Q_1$ und $Q_2$ und dem Mischungsverhältnis v werden die Anteile der ersten und zweiten Hauptkomponente in dem Zielproduktstrom, ausgedrückt als Qualität $Q_3$, als Ist-Wert berechnet.

Schließlich wird in Abhängigkeit der Abweichung des Ist-Werts von dem Zielwert für den Anteil der ersten Hauptkomponente in dem dritten Sumpfproduktstrom die Zufuhr von thermischer Energie in den Verdampfer verändert.

Die erste und zweite Hauptkomponente können einzelne Verbindungen sein. Es ist auch möglich, dass eine Mehrzahl von Verbindungen mit ähnlichen Siedetemperaturen wie ein Stoff behandelt wird und diese Mehrzahl dann die Hauptkomponente bildet.

Der Verdampfer wird häufig auch als "Reboiler" bezeichnet. Als thermische Trennvorrichtung kommen unter anderem Kolonnen wie Destillationskolonnen oder wie Rektifikationskolonnen oder aber Apparate zur Entspannungsverdampfung in Betracht, ohne jedoch darauf beschränkt zu sein.

Zur Verbesserung der Prädiktion des Softsensors kann das thermodynamische Modell verfeinert werden. Bei bekanntem nicht-idealen Verhalten des Gemisches kann die Nichtlinearitäten der Flüssigphase über die Berechnung von Aktivitätskoeffizienten über ein geeignetes $G^E$-Modell (Exzessgröße), zum Beispiel einem NRTL-Ansatz (Non-Random-Two-Liquid-Modell), berücksichtigt werden. Nichtidealitäten der Gasphase können über eine Berechnung der Fugazitätskoeffizienten mittels einer geeigneten Zustandsgleichung (zum Beispiel kubische Peng-Robinson oder PC-SAFT) berücksichtigt werden.

Gemäß einer weiteren Ausführungsform sind daher das erste und/oder das zweite vorbestimmte thermodynamische Modell ausgewählt aus: einem Modell, dem die Clausius-Clapeyron-Gleichung zugrunde liegt; einem Modell, dem die Antoine-Gleichung zugrunde liegt, einem Non-Random-Two-Liquid-Modell, einem Universal Quasichemical-Modell oder einem Universal Quasichemical Functional Group Activity Coefficients-Modell.

In einem System zur thermischen Trennung einer Mischung umfassend eine erste Hauptkomponente und eine zweite Hauptkomponente, wobei der Siedepunkt der ersten Hauptkomponente niedriger als der Siedepunkt der zweiten Hauptkomponente ist (jeweils unter den gleichen Bedingungen), umfassend einen Rechner zur Steuerung der thermischen Trennung, ist der Rechner zur Steuerung des erfindungsgemäßen Verfahrens eingerichtet.

Wenn zur Erläuterung das erfindungsgemäße Verfahren und System zur Veranschaulichung im Zusammenhang mit der MCB-Konzentration im Herstellungsverfahren für HDI erläutert werden, ist dieses nicht als Einschränkung zu verstehen. Die entwickelte Lösung ist auch auf weitere Anwendungen, die durch ein thermodynamisches Flüssig-Dampf-Gleichgewicht beschrieben werden können, übertragbar, zum Beispiel Zweistoff- oder Quasi-Zweistoffgemische. Weitere Einsatzmöglichkeiten finden Sich unter anderem im Bereich der Isocyanate.

Gemäß einer Ausführungsform umfasst die erste Hauptkomponente ein Halogenaromat und/oder die zweite Hauptkomponente ein Polyisocyanat. Beispiele für Halogenaromaten sind Monochlorbenzol und die isomeren Dichlorbenzole. Beispiele für Isocyanate sind 1,4-Diisocyanatobutan, 1,5-Diisocyanatopentan, 1,6-Diisocyanatohexan (HDI), 2-Methyl-1,5-diisocyanatopentan, 1,5-Diisocyanato-2,2-dimethylpentan, 2,2,4- bzw. 2,4,4-Trimethyl-1,6-diisocyanatohexan, 1,10-Diisocyanatodecan, 1,3- und 1,4-Diisocyanatocyclohexan, 1,3- und 1,4-Bis-(isocyanatomethyl)-cyclohexan, 1-Isocyanato-3,3,5-trimethyl-5-isocyanatomethylcyclo-hexan (Isophorondiisocyanat, IPDI), 4,4'-Diisocyanatodicyclohexylmethan, 4-Isocyanatomethyl-1,8-octandiisocyanat (Triisocyanatononan, TIN), $\omega,\omega'$-Diisocyanato-1,3-dimethylcyclohexan (H6XDI), 1-Isocyanato-1-methyl-3-isocyanato-methylcyclohexan, 1-Isocyanato-1-methyl-4-isocyanato-methylcyclohexan, Bis-(isocyanatomethyl)-norbornan, 1,5-Naphthalen-diisocyanat, 1,3- und 1,4-Bis-(2-isocyanato-prop-2-yl)-benzol (TMXDI), 2,4- und 2,6-Diisocyanatotoluol (TDI) insbesondere das 2,4 und das 2,6-Isomere und technische Gemische der beiden Isomeren, 2,4'- und 4,4'-Diisocyanatodiphenylmethan (MDI), 1,5-Diisocyanatonaphthalin, 1,3-Bis(isocyanato-methyl)benzol (XDI). Bevorzugt sind Monochlorbenzol als erste Hauptkomponente und 1,6-Hexamethylendiisocyanat (HDI) als zweite Hauptkomponente.

Mit dem erfindungsgemäßen Verfahren und System kann eine kontinuierliche Bestimmung der MCB-Konzentration mit Hilfe eines Softsensors (das Softsensorkonzept beinhaltet die indirekte Bestimmung einer qualitätsrelevanten Größe über einfache Sensoren und einem bekannten physikalischen Zusammenhang) realisiert werden, der auf einem Flüssig-Dampf-Gleichgewicht der Komponenten basiert und jeweils auf der Temperaturmessung und der Druckmessung im Sumpf der Rektifikationskolonne und im Verdampfer beruht. Der Sumpfproduktstrom der Verdampfers und der Sumpfproduktstrom der Trennrichtung werden zusammengeführt. Über eine Massenbilanz und/oder über eine Energiebilanz am Mischpunkt können das Mischverhältnis der Ausgangsströme aus Sumpf und Verdampfer und die MCB-Konzentration des finalen Ausgangstroms ermittelt werden. Über eine weitere Energiebilanz über den Verdampfer und das Verhältnis der Verdampfungsenthalpien der ersten Komponente zu der des Dampfstroms kann zusätzlich der Sumpfstrom des Verdampfers berechnet werden und somit eine Temperatur im Verdampfer oder in der Trenneinheit rekonstruiert werden, welche dann nicht messtechnisch erfasst muss. Dies ist vorteilhaft, da ein Sensor ein Eingriff in den Apparat darstellt.

Die Korrelation zwischen dem Druck, der Temperatur und der molaren MCB-Konzentration für ein MCB/HDI Gemisch, das teilweise verdampft ist und sich im thermodynamischen Gleichgewicht befindet, kann grundsätzlich über die Rekonstruktion des gemessenen Drucks anhand der Summe der Partialdrücke von MCB und HDI in der Dampfphase über das Dalton-Gesetz beschrieben werden. Der Partialdruck der jeweiligen Komponente wiederum ist proportional zur molaren Flüssigkonzentration, des Dampfdrucks und des Aktivitätskoeffizienten. Die prinzipielle Beziehung zwischen dem Dampfdruck einer Komponente und der gemessenen Temperatur lässt sich über einen Antoine-Ansatz beschreiben. Die Aktivitätskoeffizienten lassen sich zum Beispiel über ein $G^E$-Modell beschreiben und die Fugazitätskoeffizienten anhand einer Zustandsgleichung. Mit Hilfe der molaren Massen und der berechneten molaren Konzentration kann dann die Massenkonzentration von MCB in der Flüssigkeit berechnet werden.

Der erfindungsgemäße Vorteil liegt unter anderem darin, dass ein allgemeines/generisches VLE-Modul zur Berechnung der stofflichen Zusammensetzung für ein konkretes Prozessleitsystem entwickelt wurde, welches auch für weitere stoffliche Anwendungen übertragen werden kann. Hierfür müssen lediglich die Dampfdrücke der jeweiligen Reinstoffe als Antoine-Ansatz abhängig von der Temperatur und die molaren Massen bekannt sein und Temperatur- und Druckmessung an der Stelle des Prozesses, der sich Im VLE befindet, verfügbar sein oder indirekt, beispielsweise über Massen- und Energiebilanzen, bestimmt werden. Sofern die Stoffdaten korrekt sind, ist kein weiterer Auslegungs- oder Implementierungsaufwand notwendig.

Das Softsensor-Konzept liefert einen zuverlässigen, kontinuierlich vorliegenden Schätzwert der MCB-Konzentration, der dem Anlagenfahrer auf dem Prozessleitsystem angezeigt wird und zur Steuerung des Prozesses eingesetzt werden kann. Die Regelung der MCB-Konzentration ersetzt vorzugsweise die Temperaturregelung der entsprechenden Anlage (die bestehende Temperaturregelung kann allerdings als Back-up verbleiben, um automatisch bei einem schlechten Status des Softsensors aktiviert zu werden).

Der Einsatz des Softsensors ermöglicht es, die Konzentration von MCB in Bereichen zu fahren, die für bestehende Anlagen neu sind (zum Beispiel 30% statt 5% oder 0.1% statt 5%). Eventuelle Umbauten der Anlage können durch größere Apparaturen (Verdampfer, Kondensator, Reaktor, Rektifikationskolonne, ...) mit beispielsweise höherer Verdampferleistung oder Kondensatorleistung oder aber größeren Rohrleitungen erreicht werden.

Im erfindungsgemäßen Verfahren kann eine Trenderfassung vorgesehen sein. Wenn ein Trend für eine Prozessgröße einen Grenzwert (Absolutwert oder Veränderung) überschreitet, kann eine Alarmierung und/oder eine Anpassung des Zielwertes für die Anteile der ersten und zweiten Hauptkomponente erfolgen. Als Prozessgrößen hierfür kommen Druck, Temperatur und Konzentration der ersten Hauptkomponente in Betracht. Die Alarmierung kann dann zu einer Anpassung der Energiezufuhr über den Verdampfer durch die Regelung der MCB-Konzentration (automatisch oder durch den Anlagenfahrer) führen, um der Störung, die durch den Trend erfasst wird, entgegenzuwirken. Zusätzlich kann der Sollwert für die Regelung angepasst werden.

Wenn in der Anlage eine Beeinträchtigung vorliegt, kann der Softsensor (inklusive Regelung) über eine Zielwertanpassung diese kompensieren.

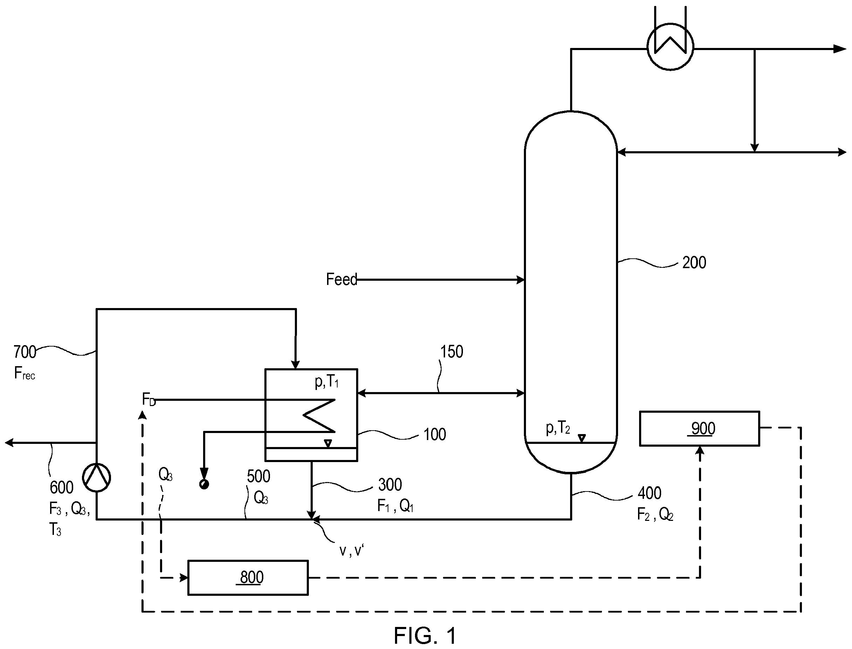

Die vorliegende Erfindung wird anhand der nachfolgenden Figur näher erläutert werden, ohne jedoch darauf beschränkt zu sein. FIG. 1 zeigt schematisch eine Anlage, in der das Verfahren durchgeführt wird.

Die Anlage weist einen Verdampfer 100 und eine thermische Trennvorrichtung 200 auf. Gemäß Schritt A) wird eine Mischung umfassend MCB (erste Hauptkomponente) und HDI (zweite Hauptkomponente) in dem Verdampfer 100 verdampft. Gemäß einer weiteren Ausführungsform wird der Verdampfer 100 mittels Dampf beheizt und der Dampf weist einen Massenstrom $F_D$ auf. Man erhält außerdem ein Sumpfprodukt.

In Schritt B) erfolgt, dargestellt durch Stoffstrom 150, ein Überführen der gasförmigen Mischung aus Schritt A) in die thermische Trennvorrichtung 200, welche beispielsweise eine Rektifikationskolonne sein kann. Das HDI ist als Schwerwieder die wesentliche Komponente des Sumpfprodukts und das MCB kann über Kopf entnommen werden. Das Überführen geschieht im einfachsten Fall passiv, das heißt durch wenigstens eine Rohrleitung, die den Verdampfer und die Trennvorrichtung fluidisch miteinander verbindet.

Gemäß Schritten C), D) und E) werden das Sumpfprodukt aus dem Verdampfer 100 im ersten Sumpfproduktstrom 300 mit einem Massenstrom $F_1$ und das Sumpfprodukt aus der Trennvorrichtung 200 im zweiten Sumpfproduktstrom 400 mit einem Massenstrom $F_2$ entfernt und zum dritten Sumpfproduktstrom 500 vereinigt. Dieser dritte Sumpfproduktstrom weist das Mischungsverhältnis v und die Qualität $Q_3$ auf.

Gemäß Schritt F) wird dieser dritte Sumpfproduktstrom 500 anschließend in den Zielproduktstrom 600, welcher dem Verfahren mit einem Massenstrom $F_3$ entnommen wird, und einen in den Verdampfer 100 mit einem Massenstrom $F_{rec}$ zurückgeführten Recycle-Strom 700 aufgeteilt. Neue zu trennende Mischung wird im Feedstrom auf die Trennvorrichtung 200 aufgegeben.

Gemäß einer weiteren Ausführungsform wird der Massenstrom $F_1$ wie folgt berechnet:

$$F_1 = F_{Rec} - F_D \cdot (h_D/h_{K1})$$

mit $h_{K1}$ als der Verdampfungsenthalpie der ersten Hauptkomponente (hier: MCB), $h_D$ als der Verdampfungsenthalpie des zum Beheizen des Verdampfers verwendeten Dampfs und $F_D$ als Massenstrom von Dampf in den Verdampfer hinein. $F_D$ und $F_{Rec}$ können hierbei über Massenstromsensoren erfasst werden.

Durch die fluidische Verbindung zwischen dem Verdampfer 100 und der Trennvorrichtung 200 herrscht dort, wo Flüssig-Dampf-Gleichgewichte vorliegen, gemeinsam der Druck p, welcher im erfindungsgemäßen Verfahren bestimmt wird. Weiterhin werden dort, wo Flüssig-Dampf-Gleichgewichte vorliegen, die Temperatur $T_1$ im Verdampfer 100 und die Temperatur $T_2$ in der Trennvorrichtung 200 bestimmt. Gemäß einer weiteren Ausführungsform wird die Tem-peratur $T_1$ mittels eines im Verdampfer 100 angeordneten Sensors gemessen. Eine weitere Ausführungsform sieht vor, dass der Druck p mittels eines im Verdampfer 100 angeordneten Sensors gemessen wird. Eine weitere Ausführungsform sieht vor, dass der Druck p mittels eines in der Trennvorrichtung 200 angeordneten Sensors gemessen wird.

Im Verfahren werden aus p und $T_1$ über ein erstes vorbestimmtes thermodynamisches Modell die Anteile von MCB und HDI in dem ersten Sumpfproduktstrom 300, ausgedrückt als Qualität $Q_1$, bestimmt.

Weiterhin werden aus p und $T_2$ über ein zweites vorbestimmtes thermodynamisches Modell die Anteile von MCB und HDI in dem zweiten Sumpfproduktstrom 400, ausgedrückt als Qualität $Q_2$, bestimmt und aus den Qualitäten $Q_1$ und $Q_2$ werden die Anteile der ersten und zweiten Hauptkomponente in dem Zielproduktstrom 600, ausgedrückt als Qualität $Q_3$, als Ist-Wert berechnet. In Abhängigkeit der Abweichung des Ist-Werts von dem Zielwert für den Anteil der ersten Hauptkomponente in dem dritten Sumpfproduktstrom 500 wird die Zufuhr von thermischer Energie in den Verdampfer 100 verändert. Dieses kann durch den Rechner 800 und das Steuerwerk 900 erfolgen. Der Rechner führt die nachfolgend beschriebenen Rechnungen durch. Durch Probenmessungen kann der Laborwert $Q_{3,Lab}$ erhalten werden, der als Kalibrierungsgröße vom Rechner verarbeitet werden kann.

In einer weiteren Ausführungsform werden der Massenstrom $F_3$ des Zielproduktstroms 600 und die in dem Zielproduktstrom 600 herrschende Temperatur $T_3$ gemessen. In Kenntnis des Massenstroms $F_3$ kann der Massenstrom $F_2$ berechnet werden: $F_2 = F_3 + F_{Rec} - F_1$.

Gemäß einer weiteren Ausführungsform wird die Temperatur $T_2$ wie folgt berechnet:

$$T_2 = [c_{p,3} \cdot T_3 \cdot (F_3 + F_{Rec}) - c_{p,1} \cdot T_1 \cdot F_1] / [c_{p,2} \cdot F_2]$$

mit $c_{p,1}$ als der Wärmekapazität des ersten Sumpfproduktstroms 300, $c_{p,2}$ als der Wärmekapazität des zweiten Sumpfproduktstroms 400 und $c_{p,3}$ als der Wärmekapazität des Zielproduktstroms 600.

Gemäß einer weiteren Ausführungsform wird die Qualität $Q_3$ mittels der Mischungsverhältnisse v oder v' wie folgt berechnet wird:

$$Q_3 = v \cdot Q_1 + (1 - v) \cdot Q_2$$

oder

$$Q_3 = v' \cdot Q_1 + (1 - v') \cdot Q_2$$

wobei v wie vorstehend definiert ist und v' wie folgt berechnet wird:

$$v' = (T_3 - T_2)/(T_1 - T_2)$$

In einer weiteren Ausführungsform werden weiterhin die Anteile der ersten und zweiten Hauptkomponente in dem Zielproduktstrom 600 wenigstens einmal experimentell bestimmt und das Ergebnis wird zur Korrektur der Berechnung der Qualität $Q_3$ verwendet.

In einer weiteren Ausführungsform wird ein Korrekturwert (Druck-Bias, $p_{bias}$) zu p addiert. Um auch zeitliche Veränderungen insbesondere der Druckmessung selber zu erfassen, kann zusätzlich eine Rückführung des Laborwerts eingeführt werden. Daher wird gemäß einer weiteren Ausführungsform der Korrekturwert, der zu p addiert wird, aus dem zuvor erwähnten experimentell bestimmten Wert berechnet. Diese Rückführung kann in Form einer Korrektur der Druckmessung p umgerechnet werden und reduziert die Diskrepanz zwischen Prädiktion und Laborwert der MCB-Konzentration zu einem Großteil.

Fehler, die sich linear zum Druck verhalten, können so vollständig kompensiert werden. Um Rauschen in der Fehlerrückführung (bedingt durch Messrauschen im Analysenlabor, Temperatur oder Druck) zu filtern, kann ein Filter angewendet werden. Dieses glättet den berechneten Druckfehler im Falle eines Filters erster Ordnung (PT1) beziehungsweise eliminiert grobe Ausreißer im Falle eines Median-Filters. Details zur Berechnung des Druck-Bias finden sich in Gleichungen (13), (14) im weiteren Verlauf dieses Textes.

Alternativ oder zusätzlich zur Verwendung des Druck-Bias können anhand der Labor- und Sensordaten beide Aktivitätskoeffizienten der Partialdrücke über eine Schätzung auf bewegtem Horizont online geschätzt werden. Details finden sich in Gleichung (15) im weiteren Verlauf dieses Textes. Da beide Aktivitätskoeffizienten linear in die Berechnung eingehen, lässt sich die Parameter-Schätzung analytisch lösen und ebenfalls in einem Prozessleitsystem mit einfachen Mitteln implementieren. Durch die Anpassung der Aktivitätskoeffizienten für verschiedene Konzentrationsbereiche wird somit der Prädiktions-Fehler des Softsensors weiter reduziert. Nichtidealitäten der Gasphase können analog durch Bestimmung der Fugazitätskoeffizienten berücksichtigt werden.

In einer weiteren Ausführungsform werden daher unter Verwendung von p, $T_1$ und/oder $T_2$ sowie des zuvor erwähnten experimentell bestimmten Wertes die Aktivitätskoeffizienten der Partialdrücke der ersten Hauptkomponente und/oder der zweiten Hauptkomponente in einer Zustandsschätzung basierend auf einem Kalman-Filter oder einer Least-Squares Parameterschätzung auf einem bewegten Horizont abgeschätzt.

Um ein robustes und zuverlässiges Signal der MCB-Konzentration zu erhalten, ist es vorteilhaft, den Status des Temperatur- beziehungsweise des Drucksensors auf den Ausgang des Softsensors zu propagieren. Zusätzlich können jeweils sowohl ein Arbeitsbereich als auch eine maximale Änderungsrate für Temperatur- beziehungsweise Drucksensor definiert werden, die bei Verletzung den Status des Softsensors auf "schlecht" schalten. In einer weiteren Ausführungsform wird daher weiterhin der Betriebsstatus von eingesetzten Sensoren überwacht und es wird bei Erfüllen von vorbestimmten Kriterien auf ein Alternativverfahren gewechselt. Auf diese Weise kann die Destillationsanlage auch bei Sensorversagen sicher betrieben werden. Das Alternativverfahren kann das herkömmliche laborbasierte Verfahren zur Bestimmung des MCB-Gehalts sein oder aber eine Temperaturregelung sein.

Gemäß einer weiteren Ausführungsform ist das Verfahren ein kontinuierliches Verfahren.

Am Beispiel der Zweistoffmischung MCB/HDI soll erläutert werden, wie der Molenbruch von MCB in der Flüssigphase berechnet werden kann. Aus dem Raoult'schen Gesetz ergibt sich:

$$y_{MCB} \cdot \Phi_{MCB} \cdot p = x_{MCB} \cdot \gamma_{MCB} \cdot p^*_{MCB} \qquad (1)$$

($y_{MCB}$: Molenbruch von MCB in der Gasphase; $\Phi_{MCB}$: Fugazitätskoeffizient von MCB; $p$: Gesamtdruck; $x_{MCB}$: Molenbruch von MCB in der Flüssigphase; $\gamma_{MCB}$: Aktivitätskoeffizient von MCB; $p^*_{MCB}$: Sättigungsdampfdruck von MCB)

Der Dampfdruck $p^*_{MCB}$ der Reinstoffkomponente hängt von der Temperatur T (hier in °C) ab und kann unter anderem nach Antoine mit den Parametern A, B und C berechnet werden:

$$p^*_{MCB} = \exp(A_{MCB} + B_{MCB}/(C_{MCB} + 273.15 + T)) \tag{1b}$$

Gleichung (1) umgeformt nach $p$ ergibt:

$$p = \frac{x_{MCB} \cdot \gamma_{MCB} \cdot p^*_{MCB}}{y_{MCB} \cdot \Phi_{MCB}} \tag{2}$$

Der Druck p ist die Summe der Partialdrücke:

$$p = p_{MCB} + p_{HDI} \tag{3}$$

($p_{MCB}$: Partialdruck von MCB in der Gasphase; $p_{HDI}$: Partialdruck von HDI in der Dampfphase)
Die Partialdrücke lassen sich für ideale Fugazitätsfaktoren $\Phi_{MCB}$ un $\Phi_{HDI}$ wie folgt formulieren:

$$p_{MCB} = x_{MCB} \cdot \gamma_{MCB} \cdot p^*_{MCB} \tag{4}$$

$$p_{HDI} = x_{HDI} \cdot \gamma_{HDI} \cdot p^*_{HDI} \tag{5}$$

In der Zweistoffmischung kann man $x_{MCB} + x_{HDI} = 1$ ansetzen. Dann kann man den HDI-Partialdruck wie folgt formulieren ( $p^*_{HDI}$: Sättigungsdampfdruck von HDI):

$$p_{HDI} = (1 - x_{MCB}) \cdot \gamma_{HDI} \cdot p^*_{HDI} \tag{6}$$

Wenn man die Ausdrücke für die Partialdrücke in die Gleichung (3) einsetzt, erhält man:

$$p = (x_{MCB} \cdot \gamma_{MCB} \cdot p^*_{MCB}) + ((1 - x_{MCB}) \cdot \gamma_{HDI} \cdot p^*_{HDI}) \tag{7}$$

$$p = (x_{MCB} \cdot \gamma_{MCB} \cdot p^*_{MCB}) + ((\gamma_{HDI} - x_{MCB} \cdot \gamma_{HDI}) \cdot p^*_{HDI}) \tag{8}$$

$$p = (x_{MCB} \cdot \gamma_{MCB} \cdot p^*_{MCB}) + (\gamma_{HDI} \cdot p^*_{HDI} - x_{MCB} \cdot \gamma_{HDI} \cdot p^*_{HDI}) \tag{9}$$

Auflösen nach $x_{MCB}$ ergibt:

$$p - \gamma_{HDI} \cdot p^*_{HDI} = x_{MCB} \cdot \gamma_{MCB} \cdot p^*_{MCB} - x_{MCB} \cdot \gamma_{HDI} \cdot p^*_{HDI} \tag{10}$$

$$p - \gamma_{HDI} \cdot p^*_{HDI} = x_{MCB} \cdot (\gamma_{MCB} \cdot p^*_{MCB} - \gamma_{HDI} \cdot p^*_{HDI}) \tag{11}$$

$$\frac{p - \gamma_{HDI} \cdot p^*_{HDI}}{\gamma_{MCB} \cdot p^*_{MCB} - \gamma_{HDI} \cdot p^*_{HDI}} = x_{MCB} \tag{12}$$

Auf der linken Seite der Gleichung sind nur Terme, die gemessen werden können, aus der Temperatur berechnet werden können oder aus Datenbanken erhalten/simuliert werden können.
Mit den molaren Massen $MW_{MCB}$ und $MW_{HDI}$ erhält man die Massenkonzentration $x_{Masse,MCB}$:

$$x_{Masse,MCB} = \frac{x_{MCB} \cdot MW_{HDI}}{x_{MCB} \cdot MW_{HDI} + x_{HDI} \cdot MW_{HDI}} \tag{12b}$$

Die Berechnung des Korrekturwerts für den Druck $p_{bias}$ ergibt sich aus der Abweichung zwischen dem einem berechneten Druckwert $p_{calc}$, der der Laboranalyse $x_{MCB}$ entspricht, und dem vom Sensor gemessenen Drucks p:

$$p_{calc,i} = \left(x_{MCB,labor,i} \cdot \gamma_{MCB,i} \cdot p^*_{MCB,i}\right) + \left(\gamma_{HDI,i} \cdot p^*_{HDI,i} - x_{MCB,labor,i} \cdot \gamma_{HDI,i} \cdot p^*_{HDI,i}\right)$$

$$\tag{13}$$

der $p_{bias}$ wird als Median der Differenzen $p_{calc,i}$ und $p_{sensor,i}$ ($p_{sensor}$ bezeichnet den vom Sensor gemessenen Druck, i bezeichnet den Index der Laborprobe) über einen Horizont der Länge n+1 berechnet, wobei k die zuletzt analysierte Laborprobe bezeichnet:

$$p_{bias} = median\left( p_{calc,k} - p_{sensor,k}, p_{calc,k-1} - p_{sensor,k-1}, \dots, p_{calc,k-n} - p_{sensor,k-n}\right)$$

$$\tag{14}$$

Somit lässt sich der gemessene Wert $p_{sensor}$ mit $p_{bias}$ sich als Eingangswert für den Druck p in Gleichung (12) korrigieren:

$$p = p_{sensor} + p_{bias} \tag{14b}$$

Alternativ zum Median Filter kann die Berechnung des Korrekturwerts für den Druck $p_{bias}$ auch über ein Filter erster (PT1) oder höherer Ordnung erfolgen. Ein PTI-Filter kann zeitdiskret folgendermaßen unter anderem wie folgt umgesetzt werden mit $\Delta t$ als Abtastzeit für die Probennahme für die Laboranalyse und $T_{Filter}$ als Filterzeit:

$$p_{bias,k} = \frac{\Delta t}{T_{filter} + \Delta t} * \left( p_{calc,k} - p_{sensor,k}\right) + \frac{T_{filter}}{T_{filter} + \Delta t} * p_{bias,k-1}, \tag{14c}$$

wobei $p_{bias,k}$ der aktuelle Korrekturwert und $p_{bias,k-1}$ der vorherige Korrekturwert ist. In einer weiteren Ausführungsform können Parameter der Berechnung an die während der laufenden Produktion gewonnen Daten anpassen. So können unter anderem die Aktivitätskoeffizienten $\gamma_{MCB}$ und $\gamma_{HDI}$ über unter anderem ein Least-Squares Verfahren geschätzt werden um die Prädiktion des oben beschrieben Verfahrens an die Labordaten $x_{MCB,Labor,i}$ anzupassen, basierend auf Daten über einen Horizont der Länge n+1, wobei Index *k* die zuletzt analysierte Laborprobe bezeichnet:

$$\min_{\gamma_{MCB}, \gamma_{HDI}} \sum_{i=k-n}^{k} \left(x_{MCB,Labor,i} - x_{MCB,calc,i}\right)^2 \tag{15}$$
$$unter\ Bedingung\ von\ (1) - (13)$$

Die geschätzten Aktivitätskoeffizienten können für ein nicht-ideales Verhalten des Flüssig-Dampf Gemisches abhängig von Temperatur und Stoffzusammensetzung variieren. Mit dieser Vorgehensweise lässt sich somit das nicht-ideale Verhalten der Mischung abhängig von den Bedingungen (Temperatur, Druck) erfassen.
Die Berechnung des Korrekturwerts bzw. der Aktivitätskoeffizienten erfolgt sobald eine neue Laborprobe ausgewertet wurde, also mit der Taktzeit der Probennahme. Die Länge des Horizonts bzw. die Filterzeit $T_{filter}$ entspricht typischweise 10-50 Probennahmen.

**Patentansprüche**

**1.** Verfahren zur thermischen Trennung einer Mischung umfassend eine erste Hauptkomponente und eine zweite Hauptkomponente, wobei der Siedepunkt der ersten Hauptkomponente niedriger als der Siedepunkt der zweiten Hauptkomponente ist, umfassend die Schritte:

A) Verdampfen einer Mischung der ersten Hauptkomponente und der zweiten Hauptkomponente in einem Verdampfer (100) durch Zufuhr von thermischer Energie unter Erhalt einer gasförmigen Mischung der ersten Hauptkomponente und der zweiten Hauptkomponente und eines Sumpfprodukts, welche sich in einem Flüssig-Dampf-Gleichgewicht miteinander befinden;

B) Überführen der gasförmigen Mischung aus Schritt A) in eine thermische Trennvorrichtung (200), wobei in der Trennvorrichtung die zweite Hauptkomponente wenigstens teilweise als Sumpfprodukt kondensiert, die erste Hauptkomponente wenigstens teilweise in der Gasphase verbleibt und wobei ein Flüssig-Dampf-Gleichgewicht zwischen dem Sumpfprodukt und der Gasphase herrscht;

C) Entfernen des flüssigen Sumpfprodukts aus dem Verdampfer (100) in einem ersten Sumpfproduktstrom (300) mit einem Massenstrom $F_1$;

D) Entfernen des flüssigen Sumpfprodukts aus der Trennvorrichtung (200) in einem zweiten Sumpfproduktstrom (400) mit einem Massenstrom $F_2$;

E) Vereinigen des ersten (300) und des zweiten (400) Sumpfproduktstroms zu einem dritten Sumpfproduktstrom (500) mit einem Mischungsverhältnis $v = F_1/(F_1 + F_2)$;

F) Aufteilen des dritten Sumpfproduktstroms (500) in wenigstens einen Zielproduktstrom (600) mit einem Massenstrom $F_3$ und einen Recycle-Strom (700) mit einem Massenstrom $F_{rec}$, wobei der Zielproduktstrom (600) entnommen wird und der Recycle-Strom (700) in den Verdampfer (100) zurückgeführt wird und wobei der Zielproduktstrom (600) einen Zielwert für die Anteile der ersten und zweiten Hauptkomponente aufweist;

**dadurch gekennzeichnet, dass**
in dem Verdampfer (100) und/oder der Trennvorrichtung (200) der dort gemeinsam herrschende Druck p bestimmt wird;
in dem Verdampfer (100) die dort herrschende Temperatur $T_1$ bestimmt wird;
in der Trennvorrichtung (200) die dort herrschende Temperatur $T_2$ bestimmt wird;
aus p und $T_1$ über ein erstes vorbestimmtes thermodynamisches Modell die Anteile der ersten und zweiten Hauptkomponente in dem ersten Sumpfproduktstrom (300), ausgedrückt als Qualität $Q_1$, bestimmt werden,
aus p und $T_2$ über ein zweites vorbestimmtes thermodynamisches Modell die Anteile der ersten und zweiten Hauptkomponente in dem zweiten Sumpfproduktstrom (400), ausgedrückt als Qualität $Q_2$, bestimmt werden,
aus den Qualitäten $Q_1$ und $Q_2$ und dem Mischungsverhältnis v die Anteile der ersten und zweiten Hauptkomponente in dem Zielproduktstrom (600), ausgedrückt als Qualität $Q_3$, als Ist-Wert berechnet werden und
in Abhängigkeit der Abweichung des Ist-Werts von dem Zielwert für den Anteil der ersten Hauptkomponente in dem dritten Sumpfproduktstrom (500) die Zufuhr von thermischer Energie in den Verdampfer (100) verändert wird.

**2.** Verfahren gemäß Anspruch 1, wobei die erste Hauptkomponente ein Halogenaromat ist und/oder die zweite Hauptkomponente ein Polyisocyanat umfasst.

**3.** Verfahren gemäß Anspruch 1 oder 2, wobei der Verdampfer 100 mittels Dampf beheizt wird und der Dampf einen Massenstrom $F_D$ aufweist.

**4.** Verfahren gemäß Anspruch 3, wobei der Massenstrom $F_1$ wie folgt berechnet wird:

$$F_1 = F_{Rec} - F_D \cdot (h_D/ h_{K1})$$

mit $h_{K1}$ als der Verdampfungsenthalpie der ersten Hauptkomponente, $h_D$ als der Verdampfungsenthalpie des zum Beheizen des Verdampfers verwendeten Dampfs und $F_D$ als Massenstrom von Dampf in den Verdampfer hinein.

**4.** Verfahren gemäß einem der Ansprüche 1 bis 3, wobei die Temperatur $T_1$ mittels eines im Verdampfer 100 angeordneten Sensors gemessen wird.

**5.** Verfahren gemäß einem der Ansprüche 1 bis 4, wobei der Druck p mittels eines im Verdampfer 100 angeordneten Sensors gemessen wird.

**6.** Verfahren gemäß einem der Ansprüche 1 bis 5, wobei der Massenstrom $F_3$ des Zielproduktstroms (600) und die in dem Zielproduktstrom (600) herrschende Temperatur $T_3$ gemessen werden.

**7.** Verfahren gemäß Anspruch 6, wobei der Massenstrom $F_2$ wie folgt berechnet wird:

$$F_2 = F_3 + F_{Rec} - F_1$$

**8.** Verfahren gemäß Anspruch 7, wobei die Temperatur $T_2$ wie folgt berechnet wird:

$$T_2 = [c_{p,3} \cdot T_3 \cdot (F_3 + F_{Rec}) - c_{p,1} \cdot T_1 \cdot F_1] / [c_{p,2} \cdot F_2]$$

mit $c_{p,1}$ als der Wärmekapazität des ersten Sumpfproduktstroms (300), $c_{p,2}$ als der Wärmekapazität des zweiten Sumpfproduktstroms (400) und $c_{p,3}$ als der Wärmekapazität des Zielproduktstroms (600).

**9.** Verfahren gemäß Anspruch 8, wobei die Qualität $Q_3$ mittels der Mischungsverhältnisse v oder v' wie folgt berechnet wird:

$$Q_3 = v \cdot Q_1 + (1 - v) \cdot Q_2$$

oder

$$Q_3 = v' \cdot Q_1 + (1 - v') \cdot Q_2$$

wobei v wie vorstehend definiert ist und v' wie folgt berechnet wird:

$$v' = (T_3 - T_2)/(T_1 - T_2)$$

**10.** Verfahren gemäß einem der Ansprüche 1 bis 9, wobei weiterhin die Anteile der ersten und zweiten Hauptkomponente in dem Zielproduktstrom 600 wenigstens einmal experimentell bestimmt werden und das Ergebnis zur Korrektur der Berechnung der Qualität $Q_3$ verwendet wird.

**11.** Verfahren gemäß einem der Ansprüche 1 bis 10, wobei ein Korrekturwert zu p addiert wird.

**12.** Verfahren gemäß Anspruch 10 und 11, wobei der Korrekturwert, der zu p addiert wird, aus dem gemäß Anspruch 10 experimentell bestimmten Wert berechnet wird.

**13.** Verfahren gemäß Anspruch 10, wobei unter Verwendung von p, $T_1$ und/oder $T_2$ sowie des gemäß Anspruch 10 experimentell bestimmten Wertes die Aktivitätskoeffizienten der Partialdrücke der ersten Hauptkomponente und/oder der zweiten Hauptkomponente in einer Zustandsschätzung basierend auf einem Kalman-Filter oder einer Least-Squares Parameterschätzung auf einem bewegten Horizont abgeschätzt werden.

**14.** Verfahren gemäß einem der Ansprüche 1 bis 13, wobei weiterhin der Betriebsstatus von eingesetzten Sensoren überwacht wird und bei Erfüllen von vorbestimmten Kriterien auf ein Alternativverfahren gewechselt wird.

**15.** System zur thermischen Trennung einer Mischung umfassend eine erste Hauptkomponente und eine zweite Hauptkomponente, wobei der Siedepunkt der ersten Hauptkomponente niedriger als der Siedepunkt der zweiten Hauptkomponente ist, umfassend einen Rechner (800) zur Steuerung der thermischen Trennung,
**dadurch gekennzeichnet, dass**
der Rechner zur Steuerung des Verfahrens gemäß einem der Ansprüche 1 bis 14 eingerichtet ist.

FIG. 1

Europäisches
Patentamt

European
Patent Office

Office européen
des brevets

# EUROPÄISCHER RECHERCHENBERICHT

Nummer der Anmeldung

EP 18 20 2307

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | DE 195 31 806 C1 (HENKEL KGAA [DE]) 10. April 1997 (1997-04-10) * Abbildung 1 * * Spalte 1, Zeile 1 - Zeile 2 * * Spalte 5, Zeile 41 - Zeile 59 * ----- | 1-15 | INV. B01D3/32 B01D3/42 C07C263/20 G05D21/00 G05B13/04 |
| Y | US 4 578 151 A (SODERSTROM III EDWIN D [US] ET AL) 25. März 1986 (1986-03-25) * Abbildung 1 * * Spalte 1, Zeile 10 - Zeile 19 * * Spalte 4, Zeile 1 - Zeile 62 * ----- | 1-15 | |
| A | EP 0 413 866 A2 (CONOCO INC [US]) 27. Februar 1991 (1991-02-27) * Abbildung 1 * * Zusammenfassung * * Beispiel 1 * ----- | 1-15 | |
| A | EP 0 555 714 A2 (BAYER AG [DE]) 18. August 1993 (1993-08-18) * Abbildung 1 * * Seite 2, Zeile 29 - Seite 3, Zeile 16 * ----- | 1-15 | RECHERCHIERTE SACHGEBIETE (IPC) |
| A | US 3 230 158 A (MOLIQUE LAWRENCE G) 18. Januar 1966 (1966-01-18) * Spalte 2, Zeile 65 - Spalte 3, Zeile 25; Abbildungen 1, 2 * ----- | 1-15 | B01D G05B C07C G05D |
| A | JP 2000 302725 A (DAICEL CHEM) 31. Oktober 2000 (2000-10-31) * Abbildung 1 * ----- | 1-15 | |

Der vorliegende Recherchenbericht wurde für alle Patentansprüche erstellt

| Recherchenort | Abschlußdatum der Recherche | Prüfer |
|---|---|---|
| Den Haag | 28. März 2019 | Van Ganswijk, J |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

.................................................................................................................

& : Mitglied der gleichen Patentfamilie, übereinstimmendes
    Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 18 20 2307

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

28-03-2019

| Im Recherchenbericht angeführtes Patentdokument | | Datum der Veröffentlichung | Mitglied(er) der Patentfamilie | | Datum der Veröffentlichung |
|---|---|---|---|---|---|
| DE 19531806 | C1 | 10-04-1997 | KEINE | | |
| US 4578151 | A | 25-03-1986 | KEINE | | |
| EP 0413866 | A2 | 27-02-1991 | EP | 0413866 A2 | 27-02-1991 |
| | | | JP | H03163193 A | 15-07-1991 |
| | | | US | 5047125 A | 10-09-1991 |
| EP 0555714 | A2 | 18-08-1993 | DE | 4203999 A1 | 19-08-1993 |
| | | | EP | 0555714 A2 | 18-08-1993 |
| | | | ES | 2118146 T3 | 16-09-1998 |
| | | | JP | H0612131 A | 21-01-1994 |
| | | | US | 5368699 A | 29-11-1994 |
| US 3230158 | A | 18-01-1966 | KEINE | | |
| JP 2000302725 | A | 31-10-2000 | JP | 4324273 B2 | 02-09-2009 |
| | | | JP | 2000302725 A | 31-10-2000 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014175962 A1